## Europäisches Patentamt

## European Patent Office

## Office européen des brevets

(19)

(11) Veröffentlichungsnummer: **0 019 644**
**B1**

(12)
# EUROPÄISCHE PATENTSCHRIFT

(45) Veröffentlichungstag der Patentschrift:
**12.09.84**

(51) Int. Cl.³: **A 61 F 1/00**

(21) Anmeldenummer: **79101595.1**

(22) Anmeldetag: **23.05.79**

(54) **Implantierbare Absperreinrichtung für natürliche oder künstliche, röhrenartige Körperorgane.**

(43) Veröffentlichungstag der Anmeldung:
**10.12.80 Patentblatt 80/25**

(45) Bekanntmachung des Hinweises auf die Patenterteilung:
**12.09.84 Patentblatt 84/37**

(84) Benannte Vertragsstaaten:
**AT BE CH DE FR GB IT LU NL SE**

(56) Entgegenhaltungen:
**DE - A - 1 541 262**
**DE - A - 2 504 302**
**DE - A - 2 614 227**
**US - A - 3 750 194**
**US - A - 3 903 894**

(73) Patentinhaber: **Trawöger, Werner, Höttinger Au 60, A-6020 Innsbruck (AT)**

(72) Erfinder: **Szinicz, Gerhard, Dr., Kohlweg 11/14, A-6020 Innsbruck (AT)**
Erfinder: **Trawöger, Werner, Höttinger Au 60, A-6020 Inssbruck (AT)**

(74) Vertreter: **Torggler, Paul, Dr., Patentanwälte Dr. Paul Torggler DDr. Engelbert Hofinger Wilhelm-Greil-Strasse 16, A-6020 Innsbruck (AT)**

## Beschreibung

Die Erfindung bezieht sich auf eine implantierbare Absperreinrichtung gemäss dem Oberbegriff des Patentanspruches 1.

Eine derartige Absperreinrichtung ist beispielsweise der DE-A-1 541 262 zu entnehmen. Sie soll Patienten Hilfe leisten und eine Erleichterung der Lebensbedingungen verschaffen, bei denen die natürliche Funktion der röhrenartigen Körperorgane des Ausscheidungsapparates bei Versagen eines Schliessmuskels nicht mehr gegeben ist. Ebenso kann sie als Verschluss künstlicher Ausgänge für den Darm oder die Harnröhre eingesetzt werden.

Die Absperreinrichtung wird in den Körper operativ eingesetzt und weist eine im Volumen veränderbare Klammer auf, die um den abzusperrenden Darm gelegt wird. Sie ist über eine Verbindungsleitung mit einem ebenfalls in den Körper eingesetzten komprimierbaren Vorratsbehälter verbunden. Die Kompression des Vorratsbehälters expandiert die Klammer, die das röhrenartige Körperorgan sperrt. Bei Aufhebung der Kompression des Vorratsbehälters wird das Druckmedium in den Vorratsbehälter zurückgeführt und der ursprüngliche Querschnitt des Körperorgans wieder erreicht.

Zur Verrastung des Behälters in der komprimierten Stellung sind den weiblichen Teil bildende, federnd ausweichende Rastteile vorgesehen, die durch den aus hakenartigen Elementen bestehenden männlichen Teil zuerst aufgeweitet werden, und anschliessend in die Ausnehmungen der hakenartigen Elemente einschnappen. Die Lösung der Verrastung wird durch einen in Komprimierungsrichtung relativ zum Behälter verschiebbaren Druckknopf bewirkt, der die federnden Rastteile ebenfalls spreizt, so dass die hakenartigen Elemente freigegeben werden.

Die relative Verschiebbarkeit des aus dem Behälter vorstehenden Druckknopfes, der natürlich ebenfalls implantiert ist, führen auf Dauer zu ungünstigen Reaktionen des umliegenden Gewebes, wie Nekrosen, Gewebeneubildungen usw.

Die weiteren in der DE-A-1 541 262 angeführten Varianten beinhalten Rückschlagventile, Bowdenzüge und motorisch betriebene Pumpen, also für eine Implantation denkbar ungünstige Bestandteile.

Die Funktionsfähigkeit der Absperreinrichtung muss auf längstmögliche Zeit sichergestellt sein, da reparaturbedingte operative Eingriffe selbstverständlich so weit als möglich vermieden werden sollen. Ist die Funktion gerade dann gestört, wenn das röhrenartige Körperorgan versperrt ist, können für den Patienten höchst gefährliche, mitunter sogar lebensbedrohende Situationen auftreten.

Die Aufgabe der Erfindung liegt nun darin, eine implantable Einrichtung der eingangs genannten Art zu schaffen, die nicht nur eine möglichst geringe Zahl von mit dem Körper direkt in Berührung kommenden körperfremden Teilen aufweist, so dass Infektionen, Nekrosen und zusätzliche Gewebeneubildungen möglichst verringert oder vermieden werden, sondern auch eine weiter erhöhte Funktionssicherheit und damit eine auf ein Minimum herabgesetzte Störungsanfälligkeit aufweist, wobei bevorzugt bei einer Blockierung der Absperreinrichtung auch eine möglichst einfache Entleerung des Sperrelementes gegeben sein soll.

Erfindungsgemäss wird diese Aufgabe durch die im kennzeichnenden Teil des Patentanspruches 1 angegebenen Merkmale gelöst. Durch die erfindungsgemässe Ausgestaltung ist die Zahl der mechanisch bewegten Teile auf die ausschliesslich im Innern des Vorratsbehälters angeordneten Eingriffselemente beschränkt. Ungünstige Reaktionen des Gewebes auf Teile, die aus dem Vorratsbehälter innerhalb des Körpers verschiebbar vorstehen, sind damit ausgeschlossen. Bei Komprimierung des Vorratsbehälters tritt der senkrecht zur Druckrichtung ausgerichtete, ausweichende, schwenkbare Stift in die herzförmige Führungsbahn an deren Spitze ein. Bei Erreichen der komprimierten Stellung wird der Stift unter Wirkung der Feder an einer Stufe an der der Eintrittsseite gegenüberliegenden Seite der Führungsbahn verrastet. Bei der nochmaligen Betätigung läuft der Stift die zweite Hälfte der Führungsbahn entlang und schliesslich wieder aus ihr an der Spitze heraus, so dass die entspannte Stellung erzielt wird. Eine weitere Stufe am Ausgang der zweiten Hälfte der Führungsbahn stellt bei der neuerlichen Komprimierung den Eintritt des Stiftes in die erste Hälfte der Führungsbahn sicher.

Die Absperreinrichtung weist an operativ einzusetzenden Hauptteilen ein Sperrelement, eine Verbindungsleitung und den als Fördereinrichtung für das Druckmedium dienenden Vorratsbehälter auf. Als Sperrelement wird vorzugsweise eine das röhrenartige Körperorgan umgebende Manschette verwendet.

Dadurch ist einerseits der operative Eingriff ebenso klein wie bei der bekannten Absperreinrichtung, die Störungsanfälligkeit jedoch wesentlich reduziert. In einer bevorzugten Ausführung ist vorgesehen, dass der Vorratsbehälter mit einem verschliessbaren Füllstutzen versehen ist, dessen Mündung direkt unter der Körperoberfläche angeordnet wird. Durch diesen kann eine allfällige Öffnung des Sperrelementes und ein Abbau des Druckes in einer für den Patienten relativ harmlosen und einfachen Weise erfolgen. Der Vorratsbehälter kann an geeigneter Stelle, beispielsweise in die Hüftregion des Patienten, direkt unter der Körperoberfläche eingesetzt sein. Wenn von der zum Körperinnern weisenden Seite des Vorratsbehälters eine Versteifungsplatte angebracht wird, kann ein Ausweichen des Behälters in den Körper vermieden werden.

Weitere bevorzugte Ausgestaltungen des Anmeldegegenstandes sind in den Unteransprüchen näher dargelegt.

Nachstehend wird nun ein bevorzugtes Ausführungsbeispiel der Erfindung anhand der Figuren der beiliegenden Zeichnungen näher beschrieben, ohne jedoch darauf beschränkt zu sein.

Die Fig. 1 zeigt schematisch die Anordnung der erfindungsgemässen Absperreinrichtung im Körper,

die Fig. 2 eine vergrösserte Schrägansicht eines Vorratsbehälters,

die Fig. 3 eine vergrösserte Schrägansicht eines Sperrelementes,

die Fig. 4 das Ausgangselement zur Bildung eines Sperrelementes,

die Fig. 5 schematisch einen Schnitt durch den Vorratsbehälter in entspannter Stellung nach der Linie II–II der Fig. 2,

die Fig. 6 die Draufsicht auf ein Mittel zur Rückführung in die entspannte Stellung,

die Fig. 7 vergrössert einen Schnitt ähnlich Fig. 5 im Detail,

die Fig. 8 eine Draufsicht auf eine Hälfte und

die Fig. 9 vergrössert eine Draufsicht auf die zweite Hälfte des Vorratsbehälters im Schnitt nach der Linie II–II der Fig. 2 in Richtung des Pfeiles A bzw. B der Fig. 5,

die Fig. 10 eine Seitenansicht in Richtung des Pfeiles C der Fig. 9 und

die Fig. 11 eine Schrägansicht in Richtung des Pfeiles D der Fig. 10.

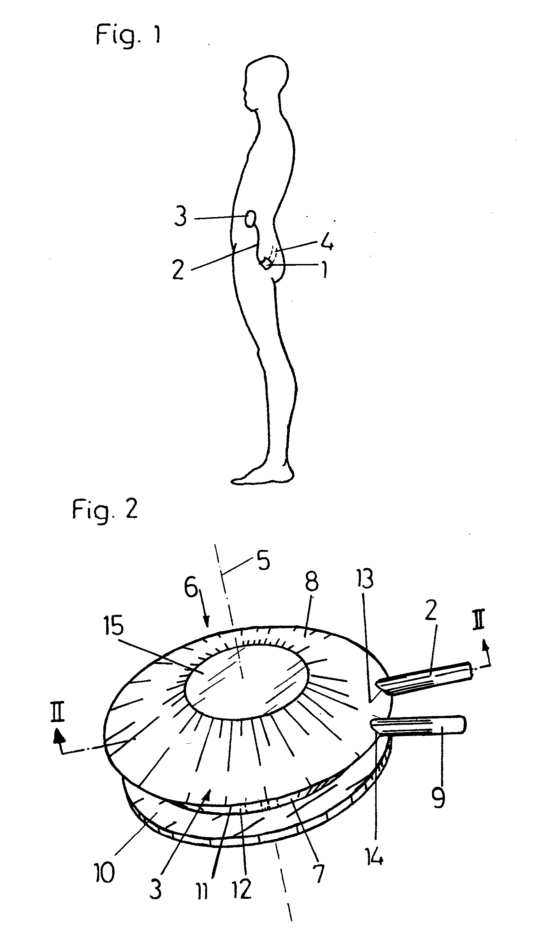

Die in Fig. 1 schematisch dargestellte Anordnung der erfindungsgemässen Absperreinrichtung zeigt deren Anwendung als künstlicher Darmverschluss. Die Absperreinrichtung besteht im wesentlichen aus drei Grundelementen, dem als Manschette 1 ausgebildeten Sperrelement, das um das röhrenartige Körperorgan 4 gelegt ist, der Verbindungsleitung 2, und dem mit einem Druckmedium gefüllten Vorratsbehälter 3. Der Vorratsbehälter 3 ist in Fig. 2 in einer vergrösserten Schrägansicht dargestellt und bildet einen ellipsoidähnlichen Rotationskörper, dessen Rotationsachse 5 in der Komprimierungsrichtung 6 liegt. Der Vorratsbehälter 3 setzt sich aus zwei Schalen 7 und 8 zusammen, die entlang ihrer Ränder 11, 12 miteinander abgedichtet verbunden sind. An einer beliebigen Stelle 13, bevorzugt im Bereich der Ränder 11, 12, mündet die Verbindungsleitung 2, bevorzugt ein Schlauch, zur Manschette 1, an einer anderen Stelle 14 mündet ein Füllstutzen 9, beispielsweise ebenfalls ein Schlauchstück, das mittels eines nichtdargestellten Verschlusses verschliessbar ist. Der Füllstutzen wird so verlegt, dass sein Verschluss unmittelbar unter der Hautoberfläche liegt. An der Schale 7 des Vorratsbehälters 3 ist eine Versteifungsplatte 10 befestigt, die beim Einsetzen in den Körper dem Körperinneren zugewendet wird, wodurch ein Nachgeben des Vorratsbehälters 3 bei Druck von aussen verhindert wird. Die Schale 8 des Vorratsbehälters 3 weist an der Aussenfläche eine zur Rotationsachse 5 koaxiale Griffmulde 15 auf, so dass die Betätigungstelle für die Volumsänderung des Vorratsbehälters 3 nach dem Einsetzen in den Körper leichter aufgefunden werden kann. Die Griffmulde 15 kann auch auf einer eigenen, auf der Schale 8 des Vorratsbehälters 3 aufgesetzten Platte ausgebildet sein.

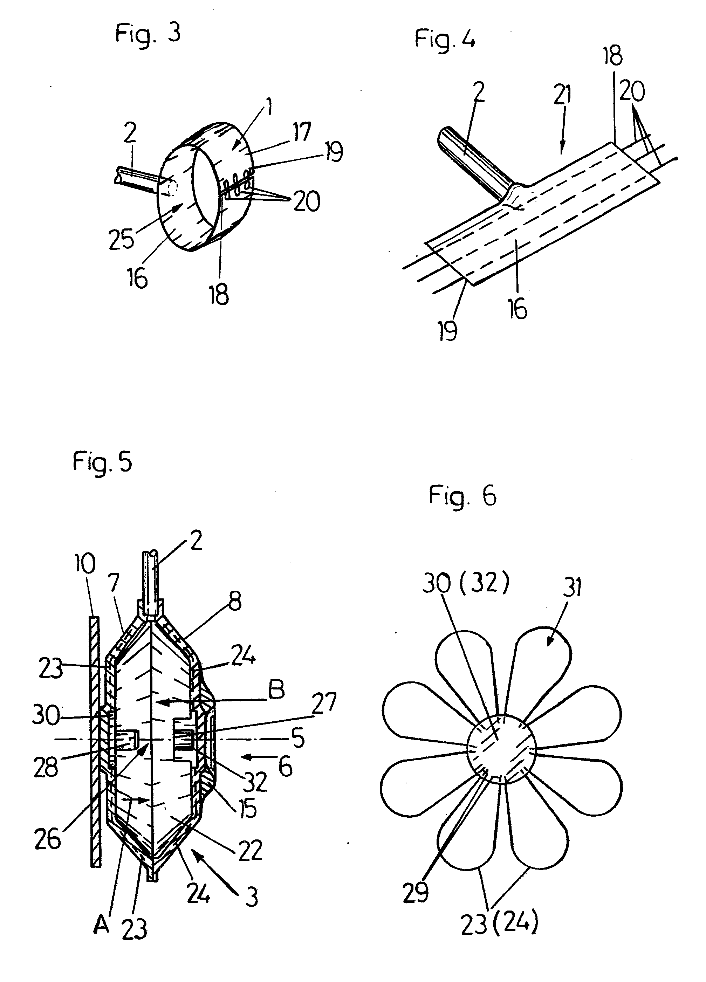

Die in Fig. 3 schematisch gezeigte Manschette 1 wird um ein röhrenartiges Körperorgan gelegt.

Die Aussenwand 17 und die Innenwand 16 der Manschette 1 schliessen eine Druckkammer ein, in die die Verbindungsleitung 2 vom Vorratsbehälter 3 mündet. Wie in Fig. 4 dargestellt, dient als Ausgangspunkt für die Manschette 1 ein aus der späteren Aussenwand 17 mit der Innenwand 16 zusammengesetzter Streifen 21, dessen Enden 18, 19 miteinander verbindbar sind, beispielsweise durch in die Aussenwand und/oder Innenwand 16, 17 eingelegte Fäden 20, die miteinander verknotet werden.

Die mit dem Körper in direkte Berührung kommenden Teile der erfindungsgemässen Absperreinrichtung bestehen aus einem zu Körpersubstanzen inerten elastischen Material oder sind mit diesem Material überzogen. Besonders geeignet ist hiezu eine unter der Bezeichnung «Silastic»® für die Firma Dow Corning Company, Midland (USA) registrierte, hochpolymere, elastische Organo-Silicium-Verbindung, die weiss, kautschukartig und wasserabstossend ist, um 70–115% gedehnt werden kann und diese Eigenschaft in einem Temperaturbereich zwischen +260 und −90 °C beibehält.

Die Fig. 5 zeigt einen Schnitt durch den Vorratsbehälter 3 parallel zur Rotationsachse 5. Die beiden Schalen 7 und 8 des Vorratsbehälters 3 für das Druckmedium sind symmetrisch zu einer Vertikalebene zur Rotationsachse 5 ausgebildet und schliessen einen Innenraum 22 ein, dessen Volumen veränderbar ist. In Fig. 5 ist der Vorratsbehälter 3 in entspannter Stellung gezeigt, das heisst, sein Innenraum 22 weist ein Volumsmaximum auf. Zur Verkleinerung des Volumens wird der Vorratsbehälter 3 in Richtung des Pfeiles 6 zusammengedrückt, wobei die Versteifungsplatte 10 als körperseitiges Widerlager dient. Als Rückstelleinrichtung 31 für den Vorratsbehälter 3 aus der komprimierten Stellung in die entspannte Stellung sind in die Wandungen der Schalen 7 und 8 Rückstellfedern 23, 24 eingebettet, von denen eine in Fig. 6 in Draufsicht gezeigt ist. Durch die Verringerung des Volumens des Innenraumes 22 wird das darin enthaltene Druckmedium über die Verbindungsleitung 2 in die Manschette 1 gedrückt, deren Durchtrittsöffnung 25 verkleinert wird. Der Querschnitt des durch die Manschette 1 geführten röhrenartigen Körperorgans 4 verengt sich und strebt gegen Null. Im Vorratsbehälter sind koaxial zur Rotationsachse 5 Eingriffselemente 26 angeordnet, die nach einer ersten Bewegung der Schale 8 aus der entspannten Stellung in Richtung des Pfeiles 6 den Vorratsbehälter 3 in komprimierter Stellung festhalten und nach einer geringfügigen zweiten Bewegung in Richtung des Pfeiles 6 die Schale 8 wieder freigeben und deren Rückführung in die entspannte Stellung mittels der Rückstellfedern 23, 24 erlauben. Dabei wird der Druck in der Manschette 1 wieder abgebaut, das Druckmedium strömt in den Vorratsbehälter 3 zurück, und der Querschnitt des abgeschnürten röhrenartigen Körperorgans 4 kann sich wieder erweitern. Die Eingriffselemente 26 zur Fixierung der komprimierten Stellung des Vorratsbehälters bestehen aus einem an der Innenseite der einen Schale 7 angeordneten männlichen Teil 28, der bei der Komprimierung in einen gegenüberliegenden weiblichen Teil 27 der Schale 8 einrastet. Der Rastmechanismus wird nachstehend noch genauer beschrieben.

Die in Fig. 6 gezeigte Rückstelleinrichtung 31 zur Rückführung des Vorratsbehälters 3 in die entspannte Stellung besteht aus einer Anzahl von etwa sternförmig angeordneten Rückstellfedern 23, 24. Diese sind etwa U-förmig gebogen, beispielsweise aus Federdraht oder geeignetem Kunststoff hergestellt, und deren Enden 29 in etwa radiale Bohrungen 35 einer Trägerplatte 30, 32 eingesetzt. Gegebenenfalls können die Rückstellfedern 23, 24 einstückig mit ihren Trägerplatten 30, 32 hergestellt sein. In die Wandung jeder Schale 7, 8 ist ein Satz Rückstellfedern 23 bzw. 24 einschliesslich der Trägerplatte 30 bzw. 32 eingebettet, wobei die Trägerplatten 30, 32 gleichzeitig auch als Trägerplatten für den weiblichen Teil 27 bzw. männlichen Teil 28 dienen. In entspannter Stellung sind die Rückstellfedern 23 bzw. 24 so gebogen, dass ihre Krümmung bzw. Wölbung der der Schalen 7, 8 des entsprechenden Vorratsbehälters 3 entspricht.

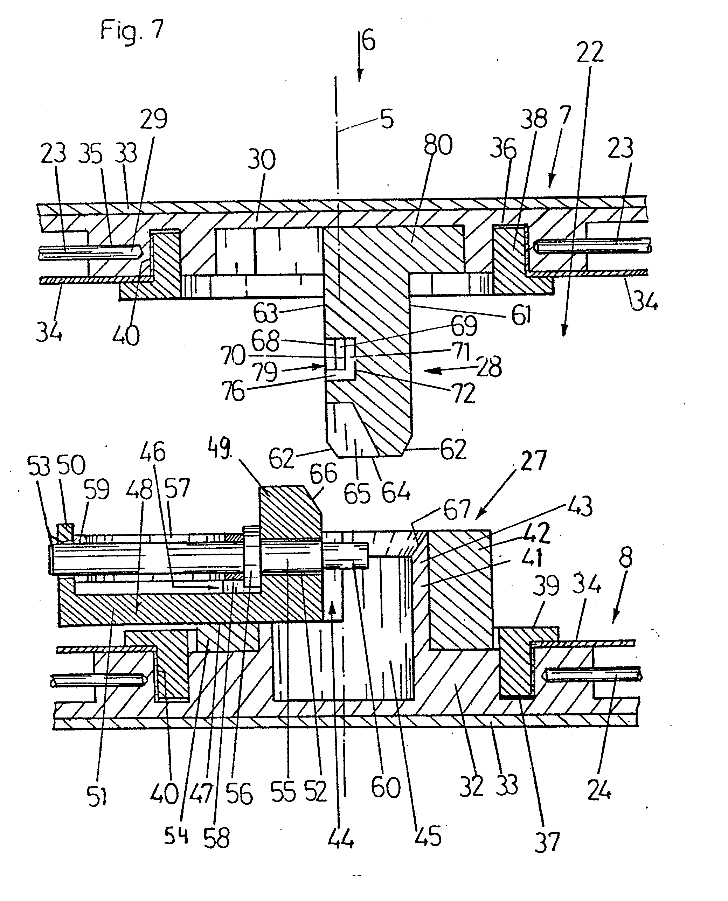

Wie aus Fig. 7 nunmehr im Detail ersichtlich, die einen Schnitt ähnlich Fig. 5 des zentralen Bereiches des Vorratsbehälters 3 zeigt, ist jede Schale 7, 8 zweischichtig ausgebildet und besteht aus einer Aussenhaut 33 und einer Innenhaut 34 aus einem elastischen zu Körpersubstanzen inerten Material, vorzugsweise aus dem bereits erwähnten Material «Silastic»®. Zwischen den Aussenhäuten 33 und den Innenhäuten 34 erstrecken sich die Rückstellfedern 23 bzw. 24. Die Aussenhäute 33 überspannen ebenfalls die Trägerplatten 30 bzw. 32, während jede Innenhaut 34 eine mit der Rotationsachse 5 konzentrische Öffnung aufweist und der Randbereich 40 in eine Ringnut 36, 37 der Trägerplatten 30, 32 eingelassen ist und dort durch einen in die Ringnut 36, 37 eingepassten Ring 38, 39 gehalten wird. Entlang der Ränder 11, 12 der Schalen 7, 8 sind die beiden Aussenhäute 33 und die beiden Innenhäute 34 miteinander dichtend verbunden, beispielsweise verklebt, verschweisst od. dgl. Das im Innenraum 22 enthaltene Druckmedium kann daher weder zwischen die Aussenhaut 33 und die Innenhaut 34 eindringen, noch aus dem Vorratsbehälter 3 an einer falschen Stelle austreten. Die dichtende Einführung der Verbindungsleitung 2 sowie des Füllstutzens 9 erfolgt vorteilhaft ebenfalls an beliebigen Stellen 13, 14 im Bereich der Ränder 11, 12 der Schalen 7, 8 ebenfalls durch Kleben oder Schweissen.

Der weibliche Teil 27 weist eine aus ihrer Trägerplatte 32 hochstehende innere Hülse 41 auf, die in einen sich etwa über 180° erstreckenden Fortsatz 43 übergeht. Der Fortsatz 43 weist demnach etwa die Form einer Hälfte einer längsgeteilten Hülse auf und lässt eine radiale Öffnung 44 in den Aufnahmeraum 45 für den männlichen Teil 28 frei. Um die innere Hülse 41 und den Fortsatz 43 ist eine äussere Hülse 42 angeordnet, die eine mit der radialen Öffnung 44 fluchtende Ausnehmung 46 aufweist. Die senkrecht zur Rotationsachse 5 verlaufende Begrenzungsfläche der Ausnehmung 46 bildet eine Auflagefläche 47 für ein etwa U-förmiges Einsatzstück 48, das mit einem die beiden seitlichen Schenkel 49, 50 verbindenden Steg 51 auf der Fläche 47 aufliegt. Die beiden seitlichen Schenkel 49, 50 sind jeweils mit einer Durchbrechung 52, 53 versehen. Der seitliche Schenkel 49 des Einsatzstückes 48 steht durch die Ausnehmung 46 und die Öffnung 44 in den Aufnahmeraum 45 für den männlichen Teil 28 vor und liegt an Schultern 54 der äusseren Hülse 42 an.

Die Durchbrechung 52 des Schenkels 49 ist als Schlitz ausgebildet, während die Durchbrechung 53 des Schenkels 50 eine Bohrung bildet. Beide dienen als Lager für einen federbeaufschlagten Stift 55 aus gehärtetem Stahl, wobei der Stift 55 aufgrund der schlitzartigen Durchbrechung 52 eine begrenzte Schwenkbewegung in einer zur Rotationsachse 5 senkrechten Ebene ausführen kann. Am Stift 55 ist zur Anlage an den Schenkel 49 eine Ringschulter 56 ausgebildet, an der sich auch eine Feder 57 abstützt. Die Feder 57 ist als elastischer Ring ausgebildet und mit Bohrungen 58, 59 versehen, die vom Stift 55 durchdrungen werden. Der diametral der Bohrung 58 gegenüberliegende Bereich der Feder 57 stützt sich am seitlichen Schenkel 50 des Einsatzstückes 48 ab. Die Feder 57 drückt also den Stift 55 in Richtung des Aufnahmeraumes 45 innerhalb der Hülsen 41, 42, wobei das vordere Ende 60 des Stiftes 55 in den Aufnahmeraum 45 ragt.

In Fig. 7 ist, wie bereits erwähnt, die entspannte Stellung des Vorratsbehälters 3 dargestellt. Dies bedeutet, dass in dieser Stellung der männliche Teil 28 nicht in den weiblichen Teil 27 eingreift, sondern die beiden Teile mit Abstand voneinander angeordnet sind.

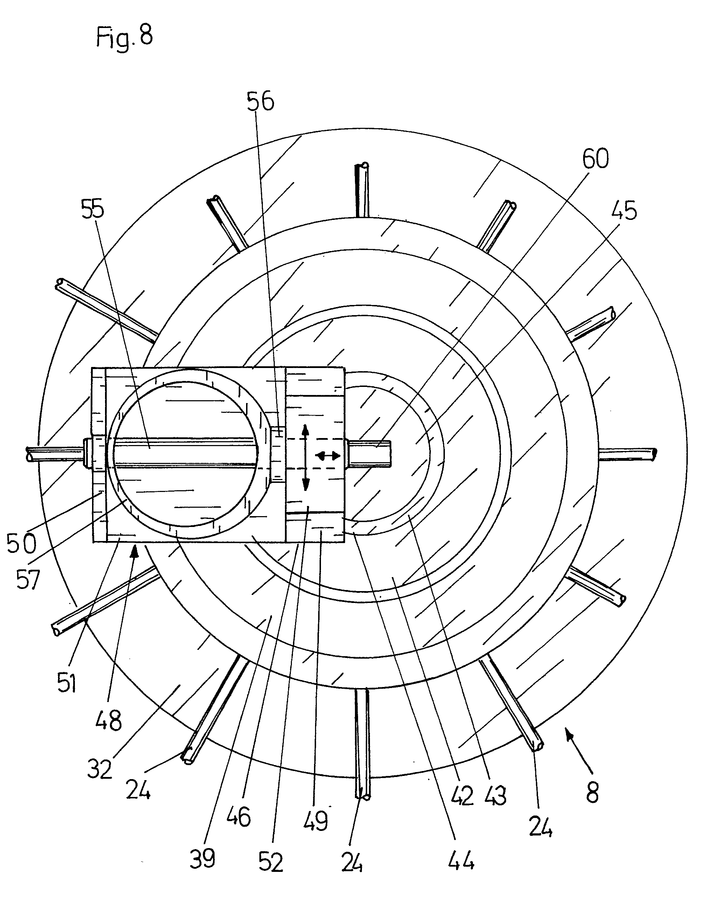

In der in Fig. 8 gezeigten Draufsicht auf den weiblichen Teil 27 und auf den zentralen Bereich der Schale 8, entsprechend dem Pfeil A in Fig. 5, wurde aus Gründen der Übersichtlichkeit die Innenhaut 34 weggelassen.

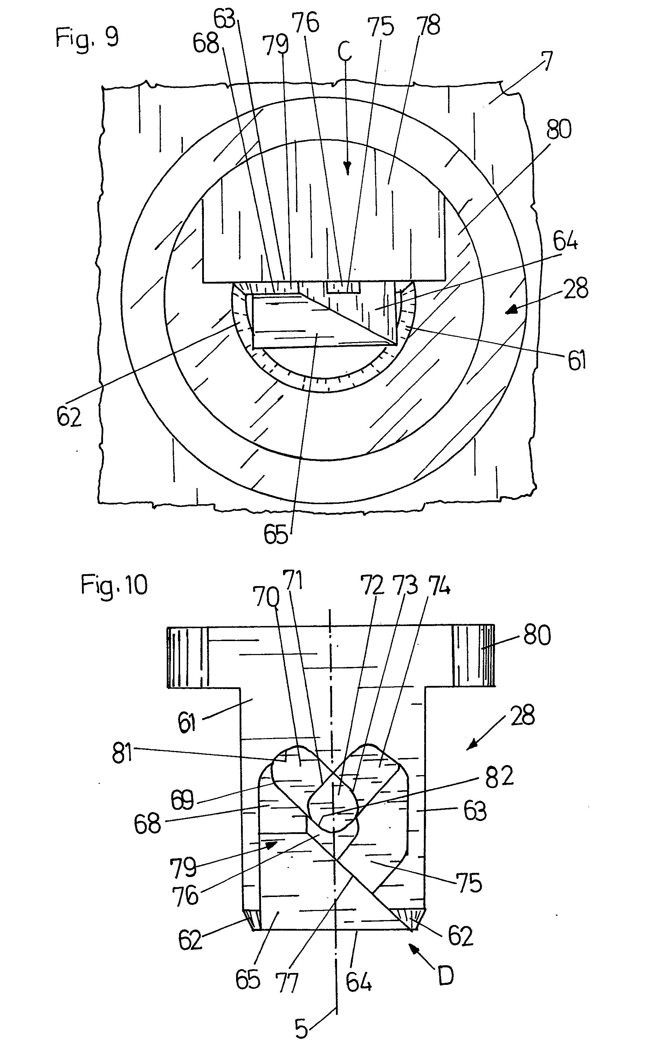

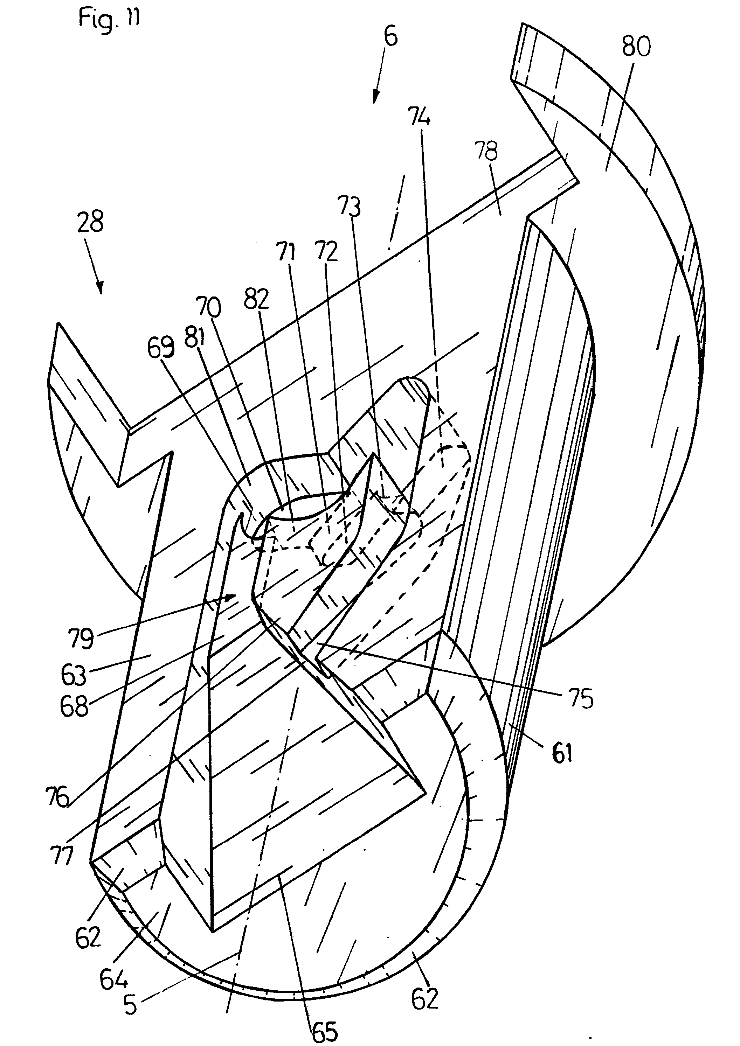

Die weitere Funktion der Eingriffselemente 26 zur Festlegung der komprimierten Stellung wird nun an Hand der Fig. 9 bis 11 näher erläutert, die verschiedene Ansichten des männlichen Teiles 28 zeigen.

Der männliche Teil 28 besteht aus einem etwa halbzylindrischen Teil 61 und einem Flansch 80, der in die Trägerplatte 30 der Schale 7 eingesetzt ist. Der halbzylindrische Teil 61 lässt eine Ausnehmung 78 für das Einsatzstück 48 des weiblichen Teiles 27 frei.

Das freie Ende 64 ist mit abgeschrägten Kanten 62 versehen. In seine ebene Fläche 63, die parallel zur Rotationsachse 5 verläuft, ist eine Führungsbahn 79 eingelassen, die das in den Aufnahmeraum 45 des weiblichen Teiles 27 ragende Ende 60 des Stiftes 55 führt. Die Führungsbahn 79 verläuft etwa herzförmig, wobei die Spitze des Herzens auf die Seite des freien Endes 64 des halbzylindrischen Teiles 61 weist und eine sich von der Spitze des Herzens zum freien Ende 64 hin erweiternde Einführung 65 aufweist. An den Wendepunkten der herzförmigen Führungsbahn 79 sind jeweils senkrecht zur Rotationsachse 5 Stufen 69, 71, 73, 77 geringer Höhe vorgesehen. Bei der Komprimierung des Vorratsbehälters 3 bewegen sich die beiden Schalen 7 und 8 in Richtung des Pfeiles 6 zueinander und der männliche Teil 28 dringt in den Aufnahmeraum 45 des weiblichen Teils 27 ein. Eine allfällige Zentrierung erfolgt mittels der abgeschrägten Kanten 62 des Teils 61 bzw. der Abschrägungen 66, 67 des inneren Schenkels 49 des Einsatzstückes 48 sowie des Fortsatzes 43. Mit fortlaufender Bewegung des männlichen Teiles 28 und damit des Teiles 61 in Richtung des Pfeiles 6 läuft das Ende 60 des Stiftes 55 auf die schräge Fläche der Einführung 65 der Führungsbahn 79 auf und wird entgegen der Kraft der Feder 57 senkrecht zur Rotationsachse 5 nach aussen verschoben. Mit fortlaufender Komprimierung gleitet das Ende 60 des Stiftes 55 entlang der ersten Führungsfläche 68 in Richtung des Flansches 80 bis zur Begrenzungswand 81 der Führungsbahn 79. Unmittelbar vor Erreichen der Begrenzungswand 81 läuft das Ende 60 des Stiftes 55 über die erste Stufe 69 und wird dabei durch die Feder 57 senkrecht zur Rotationsachse 5 bzw. ebene Fläche 63 an die zweite Führungsfläche 70 gedrückt.

Die Komprimierung des Vorratsbehälters 3 ist damit beendet und in der Manschette 1 der gewünschte Betriebsdruck geringfügig überschritten. Durch die Rückstelleinrichtung 31, die Federn 23, 24, hat der Vorratsbehälter 3 nunmehr das Bestreben, in seine entspannte Stellung zurückzukehren. Das Ende 60 des Stiftes 55 liegt jedoch an der ersten Stufe 69 an, so dass ihm der Rückweg über die erste Führungsfläche 68 verwehrt ist. Der Weg entlang der zweiten Führungsfläche 70 steht jedoch frei. Das Ende 60 des Stiftes 55 wird daher entlang der zweiten Führungsfläche 70 und entlang der seitlichen Begrenzungswand 82 des Mittelstückes 76 über die zweite Stufe 71 gleiten, worauf es durch die Feder 57 wiederum tiefer in den Teil 61 ein- und an die dritte Führungsfläche 72 angedrückt wird. Dort ist, in Komprimierungsrichtung 6 gesehen, der tiefste Punkt der Führungsbahn 79 und die Restlage für die komprimierte Stellung erzielt. Bei der Bewegung vom oberen Wendepunkt an der Begrenzungswand 81 zur Rastlage wird der Vorratsbehälter geringfügig entspannt, so dass in der Manschette 1 der richtige Betriebsdruck herrscht. Eine selbsttätige Weiterbewegung des Endes 60 des Stiftes 55 wird durch die konkave Wölbung der Begrenzungswand 82 des Mittelstückes 76 verhindert.

Soll nun der Druck in der Manschette 1 abgebaut und das röhrenartige Körperorgan geöffnet werden, wird der Vorratsbehälter 3 wiederum geringfügig komprimiert. Das Ende 60 des Stiftes 55 muss zwangsläufig entlang der dritten Führungsfläche 72 über die dritte Stufe 73 gleiten, da durch die zweite Stufe 71 der Rückweg auf die zweite Führungsfläche 70 gesperrt ist. Durch die Feder 57 wird das Ende 60 des Stiftes 55 nach Überschreiten der dritten Stufe 73 noch tiefer in den Teil 61 ein- und an die vierte Führungsfläche 74 angedrückt. In dieser Stellung wird eine weitere Komprimierung des Vorratsbehälters 3 dadurch behindert, dass das Ende 60 des Stiftes 55 an einem zweiten oberen Wendepunkt der Begrenzungswand 81 anliegt. Sobald nun die Krafteinwirkung in Richtung des Pfeiles 6 unterbrochen wird, bewirken die Rückstellfedern 23, 24 die Entspannung des Vorratsbehälters 3. Die Rückbewegung des Endes 60 des Stiftes 55 in die dritte Führungsfläche 72 ist durch die dritte Stufe 73 versperrt. Das Ende 60 des Stiftes 55 kann daher nur von der vierten Führungsfläche 74 in die mit selbem Niveau beginnende fünfte Führungsfläche 75 übergehen, über die es zurück in die Einführung 65 gleitet. Die fünfte Führungsfläche 75 steigt dabei in der Gleitrichtung des Endes 60 des Stiftes 55 an, d.h., der Stift 55 wird entgegen der Kraft seiner Feder 57 bewegt. Am Ende der fünften Führungsfläche 75 ist eine vierte Stufe 77 ausgebildet, über die das Ende 60 des Stiftes 55 auf die Einführung 65 gedrückt wird. Durch die vierte Stufe 77 wird gewährleistet, dass bei einer neuerlichen Komprimierung des Vorratsbehälters 3 das Ende 60 des Stiftes 55 auf die erste Führungsfläche 68 und nicht auf die fünfte Führungsfläche 75 gelangt. Bei der weiteren Entspannung zieht sich der Teil 61 vollständig aus dem weiblichen Teil 27 zurück, wobei das röhrenartige Körperorgan 4 freigelegt und in der Manschette vorzugsweise ein geringer Unterdruck erzeugt wird.

Die erfindungsgemässe Absperreinrichtung wird vollständig in den Körper eingesetzt, wobei die Manschette 1 um das röhrenartige Körperorgan gelegt und der Vorratsbehälter 3 mit dem Füllstutzen 9 beispielsweise in der Hüftregion direkt unter der Haut so angeordnet ist, dass eine eventuelle Änderung des Betriebsdruckes bzw. eine Behebung von Störungen im Vorratsbehälter keinen grossen operativen Eingriff bedingen. Vor allem auch die für den Patienten auftretenden Gefahren durch einen dauernden Verschluss des röhrenartigen Körperorgans bei einer Funktionsstörung der Eingriffselemente 26 zur Festlegung der komprimierten Stellung können sehr gering gehalten werden, da ein kleiner Schnitt zur Freilegung des Füllstutzens 9 genügt, um den nichtgezeigten Verschluss zu entfernen, so dass das unter Druck stehende Medium in der Manschette 1 selbsttätig ausfliessen kann, und das röhrenartige Körperorgan zumindest teilweise freigibt.

Zur Anwendung in der Humanmedizin beträgt der Volumsunterschied zwischen der entspannten und der komprimierten Stellung des Vorratsbehälters 3 zwischen 10 und 30 ml und der Druckunterschied zwischen 60 und 120 cm Wassersäule. Für die Anwendung in der experimentellen Medizin sind auch grössere Bereiche denkbar.

Als Druckmedium kann beispielsweise destilliertes Wasser verwendet werden. Weiters ist auch die Anwendung von organischen Flüssigkeiten, beispielsweise flüssigen Paraffinen denkbar. Insbesondere eignet sich dünnflüssiges Paraffin (paraffinum perli-quidum), das eine flüssige Mischung gereinigt-gesättigter Kohlenwasserstoffe darstellt. Es ist eine geruchs- und geschmacksfreie, klare, farblose, ölige und ungiftige Flüssigkeit mit der Dichte 0,83–0,87 und einer maximalen Viskosität von 65 cP.

Gegebenenfalls kann der gesamte Vorratsbehälter 3 noch von einer nichtdargestellten Aussenhülle, ebenfalls aus dem erwähnten Material «Silastic»® umgeben sein.

Ein mit einer erfindungsgemässen Absperreinrichtung laufender Dauertest verzeichnete bisher während über 100 000 Entspannungs- und Komprimierungsvorgängen nicht die geringste Funktionsstörung. Dies entspricht bei einer Zahl von durchschnittlich zwei Entspannungs- und Komprimierungsvorgängen für einen Darmverschluss pro Tag einer störungsfreien Mindestfunktionsdauer von 140 Jahren, für einen Harnröhrenverschluss bei einer durchschnittlich doppelt so hohen Betätigungszahl einer störungsfreien Mindestfunktionsdauer von 70 Jahren.

Wie bereits erwähnt, kann auch auf die Rückstelleinrichtung 31 verzichtet werden, da nach Lösen der Verrastung im Vorratsbehälter 3 – durch die unter Überdruck stehende Manschette 1 und den Durchtritt von Körpersubstanz durch die Absperrstelle – das Druckmedium rückfliesst und den Vorratsbehälter in die entspannte Stellung überführt.

**Patentansprüche**

1. Implantierbare Absperreinrichtung für natürliche oder künstliche, röhrenartige Körperorgane (4) in der Human- und/oder experimentellen Medizin, insbesondere zur Behebung der Inkontinenz, mit einem bei Zuführung eines Druckmediums den Durchtrittsquerschnitt des Körperorgans (4) verringernden Sperrelement (1), das über eine Verbindungsleitung (2) mit einem implantierbaren, aus zwei miteinander dichtend verbundenen Schalen (7, 8) bestehenden Vorratsbehälter (3) für das Druckmedium verbunden ist, dessen Volumen durch äussere Betätigung zwischen einer durch Eingriffselemente (26) an den Innenseiten der beiden Schalen (7, 8) festgelegten, komprimierten Stellung und einer durch eine Rückstelleinrichtung (31) entspannten Stellung veränderbar ist, wobei als Eingriffselemente (26) ein männlicher Teil (28) und ein weiblicher Teil (27) vorgesehen sind, die bei der Komprimierung des Vorratsbehälters (3) ineinandergeschoben werden, und an einem der beiden Teile (27, 28) ein senkrecht zur Komprimierungsrichtung (6) ausgerichteter, federbeaufschlagt ausweichender Rastteil, und im anderen Teil (27 oder 28) eine Ausnehmung (78) vorgesehen ist, in die in der komprimierten Stellung des Vorratsbehälters (3) der Rastteil eingreift, dadurch gekennzeichnet, dass als federbeaufschlagter Rastteil ein senkrecht zur Komprimierungsrichtung (6) verschwenkbarer Stift (55) vorgesehen ist, und dass die Ausnehmung (78) eine etwa herzförmige, mit Stufen (69, 71, 73, 77) in der Druckrichtung der Stiftfeder (57) versehene Führungsbahn (79) aufweist, entlang der der Stift (55) bei der Komprimierung des Vorratsbehälters (3) zwangsgeführt ist.

2. Absperreinrichtung nach Anspruch 1, dadurch gekennzeichnet, dass an jedem Wendepunkt der Führungsbahn (79) eine Stufe (69, 71, 73,77) ausgebildet ist.

3. Absperreinrichtung nach Anspruch 2, dadurch gekennzeichnet, dass die Führungsbahn (79) zumindest zwischen zwei Stufen (73, 77) entgegen der Druckrichtung der Stiftfeder (57) ansteigend verläuft.

4. Absperreinrichtung nach Anspruch 1, dadurch gekennzeichnet, dass der Stift (55) aus gehärtetem Stahl besteht.

5. Absperreinrichtung nach Anspruch 1, dadurch gekennzeichnet, dass die Rückstelleinrichtung (31) für jede Schale (7, 8) zumindest eine Rückstellfeder (23, 24) aufweist.

6. Absperreinrichtung nach Anspruch 5, dadurch gekennzeichnet, dass die Rückstellfeder (23, 24) stern- oder krallenähnlich angeordnet ist.

7. Absperreinrichtung nach Anspruch 5 und 6, dadurch gekennzeichnet, dass jede Schale (7, 8) aus einer Aussen- und einer Innenhaut (33, 34) aus elastischem Material besteht, zwischen denen die Rückstellfedern (23, 24) angeordnet sind.

8. Absperreinrichtung nach Anspruch 1, dadurch gekennzeichnet, dass der Vorratsbehälter (3) mit einem verschliessbaren Füllstutzen (9) versehen ist, dessen Mündung direkt unter der Körperoberfläche angeordnet wird.

9. Absperreinrichtung nach Anspruch 1, dadurch gekennzeichnet, dass an der beim operativen Einsetzen ins Körperinnere weisenden Schale (7) eine Versteifungsplatte (10) angeordnet ist.

10. Absperreinrichtung nach Anspruch 1, dadurch gekennzeichnet, dass an der zur Körperaussenseite weisenden Schale (8) eine Griffmulde (15) angeordnet ist.

11. Absperreinrichtung nach Anspruch 10, dadurch gekennzeichnet, dass die Griffmulde (15) an einer an der zur Körperaussenseite weisenden Schale (8) angeordneten Platte ausgebildet ist.

12. Absperreinrichtung nach Anspruch 1, dadurch gekennzeichnet, dass der Unterschied im Volumen zwischen der entspannten und der komprimierten Stellung des Vorratsbehälters (3) zwischen 10 und 30 ml beträgt.

13. Absperreinrichtung nach Anspruch 1, dadurch gekennzeichnet, dass der Druckunterschied zwischen der entspannten und der komprimierten Stellung des Vorratsbehälters (3) zwischen 40 und 200 cm Wassersäule beträgt.

**Claims**

1. Implantable closure device for natural or artificial tubular body organs (4) in human and/or experimental medicine, especially for the treatment of incontinence, comprising a blocking element (1) constricting the flow cross-section of the body organ (4) upon supply of a pressure medium and connected by means of a connecting duct (2) with an implantable supply vessel (3) for the pressure medium which consists of two sealingly joined shells (7, 8) and whose volume is by external actuation changeable between a compressed

11 0 019 644 12

position set through retaining means (26) at the internal sides of the two shells (7, 8) and an expanded position obtained by restoring means (31), with the retaining means (26) being a male part (28) and a femal part (27) which are moved into each other upon compression of the supply vessel (3), and one of the two parts (27, 28) being provided with a detent member perpendicularly aligned to the compression direction and yielding under springload and the other part (27 or 28) provided with a recess (78) into which the detent member engages in the compressed position of the supply vessel (3), characterized in that the spring-loaded detent member is a pin (55) perpendicularly pivotable to the compression direction (6), and that the recess (78) comprises a substantially heart-shaped guide track (79) which is provided with steps (69, 71, 73, 77) in the compression direction of the pin spring (57) and along which the pin (55) is compelled to move upon compression of the supply vessel (3).

2. Closure device according to claim 1, characterized in that at each inflection point of the guide track (79) a step (69, 71, 73, 77) is provided.

3. Closure device according to claim 2, characterized in that the guide track (79) extends with a rise at least between two steps (73, 77) contrary to the compression direction of the pin spring (57).

4. Closure device according to claim 1, characterized in that the pin (55) is of hardened steel.

5. Closure device according to claim 1, characterized in that the restoring means (31) has at least one restoring spring (23, 24) for each shell (7, 8).

6. Closure device according to claim 5, characterized in that the restoring spring (23, 24) is arranged in a star or spider-like manner.

7. Closure device according to claims 5 and 6, characterized in that each shell (7, 8) comprises an outer and an inner skin (33, 34) of elastic material between which the restoring springs (23, 24) are arranged.

8. Closure device according to claim 1, characterized in that the supply vessel (3) is provided with a closable filling duct (9) whose opening is arranged directly below the upper surface of the body.

9. Closure device according to claim 1, characterized in that a stiffening plate (10) is arranged at the shell (7) which is directed towards the interior of the body upon the implanting operation.

10. Closure device according to claim 1, characterized in that a hand grip (15) is arranged at the shell (8) directed towards the outer side of the body.

11. Closure device according to claim 10, characterized in that the hand grip (15) is formed at a plate disposed at the shell (8) directed towards the outer side of the body.

12. Closure device according to claim 1, characterized in that the difference in volume between the expanded and compressed positions of the supply vessel (3) lies between 10 and 30 ml.

13. Closure device according to claim 1, characterized in that the pressure differential between the expanded and compressed positions of the supply vessel (3) lies between 40 and 200 cm of water column.

**Revendications**

1. Dispositif de fermeture implantable pour organes tubulaires, naturels ou artificiels (4) du corps dans la médecine humaine et/ou dans la médecine expérimentale, notamment pour la suppression de l'incontinence, comportant un élément de blocage (1) réduisant la section transversale de passage de l'organe (4) du corps lors de l'envoi d'un fluide sous pression et qui est relié par l'intermédiaire d'une conduite de liaison (2) à un réservoir implantable (3) constitué de deux coques (7, 8), reliées entre elles de façon étanche, et prévu pour le fluide sous pression, et dont le volume peut être modifié au moyen d'un actionnement extérieur entre une position comprimée, fixée par des organes d'accrochage (26) sur les parois latérales des deux coques (7, 8), et une position de détente obtenue sous l'action d'un dispositif de rappel (31), et dans lequel il est prévu comme organes d'accrochage (26) une pièce mâle (28) et une pièce femelle (27) qui sont emboîtées l'une dans l'autre lors de la compression du réservoir (3), et qu'il est prévu sur l'une des deux pièces (26, 28) un organe d'encliquetage dirigé perpendiculairement à la direction de compression (6) et s'écartant sous l'effet d'une charge élastique, et dans l'autre pièce (26 ou 28) un évidement (78) dans lequel l'organe d'encliquetage s'engage lorsque le réservoir (3) est dans sa position comprimée, caractérisé en ce qu'il est prévu comme organe d'encliquetage chargé élastiquement, une broche (55) pouvant pivoter perpendiculairement à la direction de compression (6), et que l'évidement (78) possède un guide (78) approximativement en forme de cœur et muni d'étagements (69, 71, 73, 77) suivant la direction de poussée du ressort de broche (57) et le long duquel la broche (57) est guidée à force lors de la compression du réservoir (3).

2. Dispositif de fermeture selon la revendication 1, caractérisé en ce qu'un étagement (69, 71, 73, 77) est ménagé au niveau de chaque point d'inflexion du guide (79).

3. Dispositif de fermeture selon la revendication 2, caractérisé en ce que le guide (79) est disposé de manière à être montant à l'opposé de la direction de pression du ressort de broche (57) au moins entre deux étagements (73, 77).

4. Dispositif de fermeture selon la revendication 1, caractérisé en ce que la broche (51) est constituée par de l'acier trempé.

5. Dispositif de fermeture selon la revendication 1, caractérisé en ce que le dispositif de rappel (31) pour chaque coque (7, 8) comporte au moins un ressort de rappel (23, 24).

6. Dispositif de fermeture selon la revendication 5, caractérisé en ce que le ressort de rappel (23, 24) est réalisé en forme d'étoile ou de crampon.

7. Dispositif de fermeture selon les revendications 5 et 6, caractérisé en ce que chaque coque (7, 8) est constituée par une enveloppe extérieure

7

et une enveloppe intérieure (33, 34) constituées en un matériau élastique et entre lesquelles sont disposés les ressorts de rappel (23, 24).

8. Dispositif de fermeture selon la revendication 1, caractérisé en ce que le réservoir (3) est équipé d'une tubulure de remplissage (9) pouvant être fermée et dont l'embouchure est disposée directement au-dessous de la surface du corps.

9. Dispositif de fermeture selon la revendication 1, caractérisé en ce qu'une plaque de rigidification (10) est disposée sur la coque (7) tournée vers l'intérieur du corps lors de l'utilisation opérationnelle.

10. Dispositif de fermeture selon la revendication 1, caractérisé en ce qu'une cavité de préhension (15) est ménagée sur la coque (8) tournée vers la face extérieure du corps.

11. Dispositif de fermeture selon la revendication 10, caractérisé en ce que la cavité de préhension (15) est ménagée sur une plaque montée sur la coque (8) tournée vers la surface extérieure du corps.

12. Dispositif de fermeture selon la revendication 1, caractérisé en ce que la différence de volume entre la position détendue et la position comprimée du réservoir (3) est comprise entre 10 et 20 ml.

13. Dispositif de fermeture selon la revendication 1, caractérisé en ce que la différence de pression entre la position détendue et la position comprimée du réservoir (3) est égale à une valeur comprise entre 40 et 200 cm de hauteur de colonne d'eau.

Fig. 1

Fig. 2

Fig. 3

Fig. 4

Fig. 5

Fig. 6

Fig. 7

Fig. 8

0 019 644

Fig. 9

Fig. 10

17

Fig. 11

0 019 644